(19) Europäisches Patentamt
European Patent Office
Office européen des brevets

(11) EP 1 260 531 A1

(12) DEMANDE DE BREVET EUROPEEN

(43) Date de publication:
**27.11.2002 Bulletin 2002/48**

(51) Int Cl.[7]: **C08F 290/06**, C08F 290/04, A61K 7/48, A61K 7/06

(21) Numéro de dépôt: **02291195.2**

(22) Date de dépôt: **14.05.2002**

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **16.05.2001 FR 0106450**

(71) Demandeur: **L'OREAL**
**75008 Paris (FR)**

(72) Inventeur: **L'Alloret, Florence**
**75013 Paris (FR)**

(74) Mandataire: **Audier, Philippe André et al**
**Brevalex,**
**3, rue du Docteur Lancereaux**
**75008 Paris (FR)**

# (54) Polymères hydrosolubles à squelette hydrosoluble et à unités latérales à LCST, leur procédé de préparation, compositions aqueuses les contenant, et leur utilisation dans le domaine cosmétique

(57) Polymères hydrosolubles comprenant un squelette hydrosoluble et des unités latérales présentant dans l'eau une température inférieure critique de démixtion LCST, lesdits polymères étant susceptibles d'être obtenus par polymérisation radicalaire par précipitation de monomères hydrosolubles et de macromonomères comprenant une unité à LCST dont la température de démixtion par chauffage en solution aqueuse est de 5 à 40°C pour une concentration massique dans l'eau de 1 % de ladite unité.

Procédé de préparation de ces polymères par polymérisation radicalaire par précipitation.

Compositions aqueuses contenant ces polymères et utilisation de ces polymères et compositions, notamment dans le domaine cosmétique pour le nettoyage et/ou le maquillage et/ou le soin et/ou la protection solaire des matières kératiniques.

EP 1 260 531 A1

## Description

L'invention a trait à des polymères hydrosolubles comprenant un squelette hydrosoluble et des unités latérales à LCST présentant une température spécifique de précipitation dans l'eau.

L'invention a également trait au procédé de préparation de synthèse de ces polymères.

L'invention a enfin trait à des compositions aqueuses contenant ces polymères et à l'utilisation de ces polymères et compositions, notamment dans le domaine cosmétique.

Le domaine technique de l'invention peut être défini comme celui des polymères hydrosolubles comportant un squelette hydrosoluble et des unités latérales à LCST. On sait que ces polymères présentent, en solution aqueuse, des propriétés de gélification stimulées par une élévation de la température.

Ce comportement rhéologique original est particulièrement intéressant dans les domaines cosmétique et pharmaceutique car il permet d'obtenir des systèmes aqueux fluides à la température ambiante et gélifiés à la température du corps humain, c'est-à-dire des compositions fluides (émulsions, dispersions, lotions,...) qui gélifient à l'application.

Ce type d'application du pouvoir thermogélifiant est décrit dans le cas des solutions aqueuses et des émulsions dans les brevets WO-95/24430 (University of Washington, Hoffman) [6], US-5,939,485 (Medlogic Global Corporation, Bromberg) [7], WO-97/00275 (Gel Sciences, Inc, Bromberg) [8] et WO-98/48768 (Medlogic Global Corporation, Bromberg) [9].

Divers procédés de synthèse ont été utilisés pour obtenir ce nouveau type de polymères ; ces procédés sont les suivants :

I) Réaction de greffage de chaînes à LCST sur des macromolécules hydrosolubles, à l'aide d'un agent de couplage tel que le dicyclohexylcarbodiimide ou le chlorhydrate de 1-(3-diméthylaminopropyl)-3-éthyl carbodiimide. Les polymères obtenus ont une structure « en peigne ». Cette voie de synthèse est décrite par Hourdet (Hourdet D., L'Alloret F., Audebert R., Polymer, 1997, 38 (10), 2535-2547) [14], dans les brevets EP 583 814 [4], EP 629 649 [5] (Schlumberger Dowell, Maroy), WO-95/24430 (University of Washington, Hoffman) [6].

Cette voie de synthèse est difficilement transposable à l'échelle industrielle pour des raisons de coût (agent de couplage et réaction en milieu relativement dilué) et de toxicité (agent de couplage).

II) Copolymérisation d'un macromonomère à LCST et d'un monomère hydrosoluble en milieu homogène (eau ou solvant organique) ou en émulsion.

Cette voie de synthèse en milieu homogène est décrite par Hourdet (Hourdet D., L'Alloret F., Audebert R., Polymer, 1997, 38 (10), 2535-2547) [14], dans les brevets EP 583 814 [4], EP 629 649 [5] (Schlumberger Dowell, Maroy), WO-95/24430 (University of Washington, Hoffman) [6]. Le procédé de polymérisation réalisé en émulsion inverse est décrit dans le brevet WO-00/35961 [10] (Rhodia, Yeung). Les polymères obtenus ont une structure « en peigne ».

Au niveau industriel, le procédé de synthèse en milieu homogène nécessite l'utilisation de quantités d'eau ou de solvant importantes, la teneur en matière active dans le milieu réactionnel étant en général inférieure à 10%. Le polymère est obtenu soit en solution aqueuse à une teneur inférieure à 10%, ce qui limite son utilisation en terme de formulation ; soit en solvant organique et doit subir dans ce cas, une phase de récupération, par exemple par précipitation.

Pour la polymérisation en émulsion, les teneurs en matières actives sont plus importantes. Cependant la récupération du polymère nécessite l'introduction de quantités importantes de sel ($Na_2CO_3$, NaOH) afin d'obtenir le polymère sous forme solide ; ou bien conduit à un système aqueux contenant, après inversion de phases, le polymère et des tensioactifs.

III) Réaction de couplage entre des blocs hydrosolubles et des blocs à LCST.

Cette voie de synthèse, décrit dans le brevet WO-95/24430 (University of Washington, Hoffman) [6], conduit à des polymères multiblocs. Elle nécessite l'utilisation de blocs hydrophiles et de blocs à LCST fonctionnalisés, ce qui induit un procédé en plusieurs étapes, à savoir synthèse des deux types de blocs, puis réaction de couplage.

IV) Polymérisation radicalaire de monomères hydrosolubles en présence de dérivés oxyalkylénés.

Cette voie de synthèse, réalisée en milieu aqueux, par exemple dans les brevets US-5,939,485 [7], WO-97/00275 [8] et WO-98/48768 [9] et en émulsion inverse dans le brevet WO-00/35961 [10] est basée sur des réactions de transfert de radicaux aux chaînes oxyalkylénées et conduit à des composés de structure complexe dont il est difficile de maîtriser les propriétés en milieu aqueux.

V) Polymérisation radicalaire d'un macromonomère portant des chaînes de type $C_m(OE)_n$ et d'un monomère comportant au moins une fonction insaturée.

La demande de brevet EP-1 069 142 [15] (Clariant) décrit tout polymère hydrosoluble obtenu par polymérisation radicalaire d'un monomère hydrocarboné pouvant contenir des atomes d'oxygène, d'azote, de soufre, de phosphore, de chlore et/ou de fluor, et d'un macromonomère comprenant une unité oxyalkylénée et une unité hydrophobe de type $C_mH_{2m+1}$, où m est un nombre entier compris entre 0 et 30. Selon la revendication 4, le nombre m est compris de préférence entre 10 et 22. Les revendications 8 et 9 concernent des polymères présentant en solution aqueuse, au-delà d'une température critique, une viscosité constante ou bien un pouvoir épaississant par chauffage. Dans ce cas, les macromonomères utilisés contiennent une unité présentant dans l'eau une LCST.

Lorsque le nombre m est supérieur à 6, les greffons de ces polymères « en peigne » sont des tensioactifs alkyléthoxylés, composés connus pour présenter dans l'eau un point de trouble par élévation de la température. Les polymères greffés correspondants sont, au-dessous de ce point de trouble, de nature associative car ils portent des unités hydrophobes alkyles. Ils possèdent ainsi des propriétés gélifiantes en dessous du point de trouble de leurs greffons (voir exemple comparatif 1 pages 14 et 15 de EP-A-1,069,142). D'autre part, ces polymères à greffons alkyléthoxylés sont susceptibles d'interagir avec les tensioactifs présents dans le milieu, et particulièrement avec les amphiphiles de même nature chimique ; cette sensibilité aux tensioactifs limite la flexibilité de ces systèmes en terme de formulation. Notons par ailleurs que les propriétés thermogélifiantes données dans les exemples sont observées audelà de 90°C.

L'exemple 17 décrit un polymère portant des greffons polyoxyéthylénés (m = 0) ; son comportement rhéologique en milieu aqueux en fonction de la température n'est cependant pas décrit.

La revendication 11 précise le procédé de synthèse préféré, basé sur une polymérisation radicalaire par précipitation dans le tertiobutanol.

En conclusion, cette demande de brevet décrit une voie de synthèse simple à mettre en oeuvre du point de vue industriel, utilisant un solvant non toxique adapté aux applications cosmétiques et pour laquelle le rapport Polymère/Solvant est plus important que dans le cas des procédés réalisés en milieu homogène. Les polymères thermogélifiants décrits dans cette demande de brevet portent des chaînes thermosensibles ayant des unités oxyalkylénées ; l'ensemble des polymères décrits ont cependant une température de gélification supérieure à 90°C, peu adaptée aux applications cosmétiques.

Il existe donc un besoin pour des polymères hydrosolubles comprenant un squelette hydrosoluble portant des unités à LCST qui, entre autres :

- soient adaptés aux applications cosmétiques avec une température de précipitation des unités à 1% dans l'eau dans une plage intéressante du point de vue cosmétique ;
- dont le procédé de synthèse soit extrapolable à l'échelle industrielle tout en permettant une bonne maîtrise de la structure des macromolécules, et donc de leurs propriétés en solution et à l'état de film.

Le but de la présente invention est, entre autres, de répondre à ces besoins.

Ce but, et d'autres encore, sont atteints, conformément à l'invention par un polymère hydrosoluble comprenant un squelette hydrosoluble et des unités latérales présentant dans l'eau une température inférieure critique de démixtion LCST, ledit polymère étant susceptible d'être obtenu par polymérisation radicalaire par précipitation de :

a) un ou plusieurs monomères hydrosolubles (Ia), et, éventuellement, de un ou plusieurs monomères hydrophobes (Ib) en une quantité minoritaire par rapport aux monomères (Ia), lesdits monomères (Ia) et (Ib) portant au moins une fonction insaturée susceptible d'être polymérisée pour former le squelette hydrosoluble ;
b) un ou plusieurs macromonomères répondant à la formule (II) suivante :

$$A\text{-}X\text{-}B \qquad (II),$$

dans laquelle A est un groupe comprenant au moins une liaison hydrocarbonée insaturée susceptible d'être polymérisée ; X est un groupe choisi parmi -O-, -S-, -PH-, -NH-, et NR° où R° est un groupe alkyle de 1 à 6 atomes de carbone ; et B est une unité à LCST dont la température de démixtion par chauffage en solution aqueuse est de 5 à 40°C pour une concentration massique dans l'eau de 1 % de ladite unité.

Dans le squelette hydrosoluble, le ou les monomères hydrosolubles (Ia) sont en quantité suffisante pour permettre la solubilité dans l'eau, du squelette hydrosoluble. On entend par squelette hydrosoluble un squelette soluble dans l'eau de 5° à 80°C, à raison d'au moins 10 g/l, de préférence au moins 20 g/l.

De manière surprenante, les polymères selon l'invention, qui sont des polymères hydrosolubles portant des unités à LCST spécifiques, sont parfaitement adaptés, en particulier à une utilisation cosmétique, du fait de la température spécifique de précipitation des unités à LCST à 1 % dans l'eau, qui se situe dans la plage de 5 à 40°C.

Ces polymères, en particulier du fait de la manière dont ils sont préparés, présentent une structure parfaitement contrôlée, maîtrisée, et parfaitement définie.

En conséquence, leurs propriétés, par exemple en solution et à l'état de film, sont également parfaitement définies et contrôlées.

L'invention a également trait à un procédé de préparation du polymère, tel qu'il a été décrit ci-dessus, dans lequel :

- on réalise la copolymérisation radicalaire par précipitation d'un ou plusieurs monomères hydrosolubles (Ia), éventuellement, de un ou plusieurs monomères hydrosolubles (Ib) en une quantité minoritaire par rapport aux monomères (Ia), et d'un ou plusieurs macromonomères de formule (II) dans un milieu comprenant un alcool et de l'eau ;
- on isole le polymère obtenu.

Ce procédé permet la préparation des polymères décrits plus haut, de façon simple et rapide, par un procédé fiable et éprouvé de polymérisation radicalaire par précipitation dans un milieu hydroalcoolique.

Ce procédé comporte peu d'étapes, en fait essentiellement une seule étape et est donc rapide et peu onéreux.

L'alcool de ce milieu est, de préférence, le tertiobutanol. Ce procédé de synthèse a pour avantages, entre autres, de s'effectuer dans des milieux cosmétiquement acceptable, d'être facilement extrapolable à l'échelle industrielle, et de permettre un parfait contrôle de la structure des polymères ou macromolécules obtenues, et donc de leurs propriétés en solution et à l'état de films.

Il est à noter qu'un procédé de polymérisation radicalaire par précipitation, notamment dans le terbiobutanol, est connu du document EP-A-1 069 142, mais il n'était absolument pas évident de transposer un tel procédé, afin de préparer les polymères spécifiques selon l'invention, dont la structure est fondamentalement différente des polymères du document EP-A-1 069 142.

En effet, en particulier, les macromonomères, entrant dans la composition du polymère selon l'invention, comportent des unités à LCST dont la température de LCST spécifique est nettement plus basse, à savoir de 5 à 40°C, à 1 % dans l'eau, que celle des polymères du document EP-A-1 069 142.

Le polymère selon l'invention est un polymère comprenant un squelette hydrosoluble et des unités présentant dans l'eau une température inférieure critique de démixtion (LCST), encore appelées « unités à LCST ».

A ce propos, il est utile de rappeler que par unités à LCST, on entend des unités dont la solubilité dans l'eau est modifiée au-delà d'une certaine température. Il s'agit d'unités présentant une température de démixtion par chauffage (ou point de trouble) définissant leur zone de solubilité dans l'eau. La température de démixtion minimale obtenue en fonction de la concentration en polymère constitué uniquement d'unités à LCST est appelée « LCST » (Lower Critical Solution Température). Pour chaque concentration en polymère à LCST, une température de démixtion par chauffage est observée. Elle est supérieure à la LCST qui est le point minimum de la courbe. En dessous de cette température, le polymère est soluble dans l'eau, au-dessus de cette température, le polymère perd sa solubilité dans l'eau.

Les unités à LCST du polymère selon l'invention ont une température de démixtion par chauffage de 5 à 40°C pour une concentration massique dans l'eau de 1 % en poids desdites unités à LCST.

De préférence, la température de démixtion par chauffage en solution aqueuse des unités à LCST du polymère est de 10 à 35°C et de préférence encore de 10 à 30°C, pour une concentration massique dans l'eau de 1 % desdites unités à LCST.

Le polymère selon l'invention ayant la structure décrite plus haut avec un squelette hydrosoluble et les unités à LCST spécifiques définies ci-dessus présente en solution aqueuse, des propriétés de gélification au-delà d'une température critique, ou propriétés de thermogélification.

Ces propriétés de gélification par chauffage observées au-delà de la température de démixtion des chaînes à LCST sont décrites dans l'art antérieur, notamment dans les documents [1], [2] et [3]. Elles sont dues à l'association des chaînes à LCST au sein de microdomaines hydrophobes au-delà de leur température de démixtion, formant ainsi des noeuds de réticulation entre les chaînes principales.

Ces propriétés gélifiantes sont observées lorsque la concentration en polymère est suffisante pour permettre des interactions entre des greffons à LCST portées par des macromolécules différentes. La concentration minimale nécessaire, appelée « concentration critique d'agrégation ou CAC », est évaluée par des mesures de rhéologie : il s'agit de la concentration à partir de laquelle la viscosité d'une solution aqueuse des polymères de l'invention devient supérieure à la viscosité d'une solution du polymère équivalent ne comportant pas de chaînes à LCST.

Au-delà de la CAC, les polymères de l'invention présente des propriétés de gélification lorsque la température devient supérieure à une valeur critique appelée « température de gélification ou Tgel ». D'après les données de la littérature, un bon accord existe entre Tgel et la température de démixtion des chaînes à LCST, dans les mêmes conditions de concentrations. La température de gélification d'une solution aqueuse d'un polymère de l'invention est déterminée par des mesures de rhéologie : il s'agit de la température à partir de laquelle la viscosité de la solution d'un polymère de l'invention devient supérieure à la viscosité d'une solution du polymère équivalent ne comportant pas de chaînes à LCST.

Les polymères de l'invention se caractérisent par une température de gélification spécifique généralement de 5 à 40°C, de préférence de 10 à 35°C, et de préférence encore de 10 à 30°C, pour une concentration massique dans l'eau, par exemple égale à 2 % en poids.

Cette température de gélification spécifique permet à ces polymères de communiquer aux compositions dans lesquelles ils se trouvent incorporés, toute une série de propriétés et, en particulier, une grande variété de formes à température ambiante, et un effet texture gélifiée lors de l'application.

Des polymères comportant, à l'instar de ceux mis en oeuvre dans les compositions de l'invention, des unités ou un squelette hydrosoluble et des unités à LCST et possédant des propriétés de gélification par chauffage observées au-delà de la température de démixtion des chaînes à LCST sont décrites dans les documents déjà cités plus haut.

Le document [1] est relatif au thermoépaississement réversible de solutions aqueuses de copolymère comprenant un squelette hydrosoluble d'acide polyacrylique avec des greffons de poly(oxyde d'éthylène) (PEO).

Le document [2] concerne le comportement thermoépaississant en solution aqueuse de polymères comportant un squelette d'acide 2-acrylamido-2-méthylpropanesulfonique (AMPS) et des chaînes latérales de poly(oxyde éthylène).

De même, le document [3] décrit la thermoassociation réversible de copolymères à squelette hydrosoluble polyacrylique ou à base d'AMPS avec des greffons de POE.

Les polymères, tels que ceux mentionnés dans les documents [1], [2] et [3] trouvent, en particulier, leur application dans l'industrie pétrolière.

Ainsi, le document [4] décrit-il des polymères thermoviscosifiants à squelette hydrosoluble comportant des segments, ou portant des chaînes latérales, à LCST qui peuvent être utilisés notamment en tant qu'agents épaississants, constituants de fluides de forage ou autres fluides, et fluides de nettoyage industriel.

Le document [5] décrit des polymères analogues à ceux du document [4] et leur utilisation en tant qu'agent anti-sédimentation de suspensions, éventuellement dans les préparations cosmétiques.

Il est à noter qu'aucun des documents [1] à [5] ne décrit des polymères présentant la structure spécifique des polymères selon l'invention.

Le document [6] décrit également les copolymères comprenant un squelette constitué par des unités sensibles au pH, par exemple des unités polyacryliques, et des unités sensibles à la température, greffées sur ce squelette. Ces copolymères présentent des propriétés de gélification en température et ils sont utilisés pour la libération et le relargage contrôlé de principes actifs ou pharmaceutiques et, éventuellement cosmétiques, par application topique.

Les polymères selon le document [6] se caractérisent par l'opacité extrêmement gênante des produits obtenus en température, ce qui n'est pas le cas des polymères de l'invention.

En fait, le polymère de ce document est fondamentalement différent de celui de l'invention car il présente globalement pour l'ensemble du polymère une LCST dans la plage de températures de 20 à 40°C, contrairement aux polymères de l'invention qui sont hydrosolubles pour toute température entre 5° et 80°C.

Les documents [7] et [8] décrivent des systèmes polymères à gélification réversible, comprenant un composant sensible capable d'agrégation, en réponse à un changement dans un « stimulus » extérieur, et un composant structural. Le stimulus extérieur peut être, par exemple, la température.

Le composant sensible au stimulus extérieur est fondamentalement différent des unités à LCST de la demande. En effet, ces composants sensibles au stimulus extérieur sont en fait constitués par au moins un fragment hydrophile et un fragment hydrophobe. Ainsi, le composant sensible peut être un copolymère bloc, tel qu'un « poloxamer », par exemple un PLURONIC®, qui est un polymère bloc d'oxyde éthylène (soluble) et d'oxyde de propylène (insoluble), un tel copolymère bloc s'agrège au niveau microscopique au-delà d'une température critique ne correspondant pas à une LCST. On peut aussi utiliser comme composant sensible un tensioactif non ionique.

Le document [7] concerne plus particulièrement un réseau polymérique formé d'un squelette hydrosoluble polyacrylique et d'un composant sensible de PLURONIC®, qui est enchevêtré dans ledit squelette, sans liaison covalente, ce réseau a donc une structure particulière qui n'a rien de commun avec le polymère de l'invention. Par contre, dans le document [8], il est question de polymères avec des liaisons covalentes.

Ces polymères ont des propriétés de gélification par chauffage et ils peuvent être utilisés dans le domaine pharmaceutique pour la délivrance de médicaments et dans de nombreux autres domaines y compris le domaine des cosmétiques.

Dans ces formulations, le composant sensible du système polymère a un comportement différent de celui d'unités à LCST, telles que celles du polymère de l'invention, lors du chauffage. Ainsi, lorsqu'on chauffe ledit composant sensible (par exemple, poloxamer), à environ 30-40°C, il présente une température de micellisation, c'est-à-dire une agrégation à l'échelle microscopique, puis lorsqu'on le chauffe davantage, une température de LCST très supérieure. Cette LCST correspond à une agrégation à l'échelle macroscopique entre les macromolécules. Il est expliqué dans WO-A-97 00275 [8] aux pages 16 et 17, que la gélification et la LCST sont observées à des températures qui diffèrent d'environ 70°C, la température de gélification correspondant à la température de micellisation du composant sensible, ce qui montre que ces polymères sont différents de ceux de notre demande. En outre, il n'est pas possible, du fait de

la synthèse utilisée dans le document [8], de contrôler parfaitement la structure et les propriétés du polymère final obtenu, comme c'est le cas dans les compositions de l'invention. En effet, la préparation des polymères de l'invention, par polymérisation radicalaire de macromonomères spécifiques à unités à LCST et de monomères, permet, au contraire, de contrôler, de maîtriser parfaitement la structure et les propriétés du polymère.

On connaît encore par le document [9] des compositions cosmétiques utilisant un système polymère à thermogélification réversible, comprenant l'acide polyacrylique et un poloxamer comme dans les documents [7] et [8]. De nouveau, le système polymère de ces documents est fondamentalement différent de ceux de l'invention, si bien que les propriétés avantageuses des polymères de l'invention ne peuvent être obtenues.

WO-A-00 35961 [10] décrit la préparation de polymères ayant des propriétés d'épaississement en température par polymérisation en émulsion et l'utilisation de ces polymères dans des compositions pharmaceutiques et cosmétiques. Ces polymères peuvent être des copolymères ayant des unités hydrosolubles et des unités à LCST à base d'oxyde d'alkylène. Là encore, la structure et le procédé de préparation de ces polymères sont fondamentalement différents de ceux de l'invention.

Il ressort de ce qui précède que la structure des polymères selon l'invention, présentant, en particulier, des unités à LCST spécifiques, ainsi que leur procédé de préparation par polymérisation radicalaire par précipitation dans un milieu hydroalcoolique, ne sont ni décrits ni suggérés dans les documents de l'art antérieur.

Comme on l'a indiqué plus haut, le polymère selon l'invention comprend un squelette hydrosoluble et des unités présentant dans l'eau une LCST, ledit polymère étant susceptible d'être obtenu par polymérisation radicalaire de un ou plusieurs monomères hydrosolubles (Ia), éventuellement de un ou plusieurs monomères hydrophobes (Ib) en une quantité minoritaire par rapport auxdits monomères (Ia), lesdits monomères (Ia) et (Ib) formant le squelette hydrosoluble, et d'un ou plusieurs macromonomères (II) comportant des unités à LCST.

Les polymères de l'invention sont donc essentiellement généralement des polymères greffés dont le squelette est hydrosoluble, ledit squelette étant porteur de greffons comprenant des unités à LCST.

Le squelette hydrosoluble ne présente pas de température de démixtion par chauffage.

Lesdits polymères peuvent être partiellement ou totalement réticulés.

Le squelette hydrosoluble comprend en majorité des unités hydrosolubles, ces unités hydrosolubles (Ia) étant issues des monomères (Ia) qui portent, avant la polymérisation, au moins une fonction insaturée.

Par unités hydrosolubles, on entend généralement que ces unités sont des unités solubles dans l'eau, à une température de 5 à 80°C, à raison d'au moins 10 g/l, de préférence d'au moins 20 g/l.

Le squelette hydrosoluble peut éventuellement également comporter une ou des unités monomères hydrophobes issues des monomères (Ib), lesdites unités hydrophobes étant éventuellement présentes, mais en une quantité minoritaire, par exemple inférieure à 20 %, c'est-à-dire en une quantité suffisamment faible, pour que le squelette hydrosoluble du polymère soit soluble dans l'eau.

Le squelette hydrosoluble ne présente pas de température de démixtion par chauffage de type LCST.

A titre d'exemple, le ou les monomères hydrosolubles (Ia) sont choisis parmi les monomères suivants ou les sels de ceux-ci :

- l'acide (méth)acrylique ;
- l'acide acrylamido-2-méthylpropane sulfonique (AMPS) ;
- l'acide styrène sulfonique ;
- l'acide vinylsulfonique ;
- l'acide (méth)allylsulfonique ;
- le (méth)acrylamide ;
- l'acide vinyl phosphonique ;
- la N-vinylacétamide ;
- la N-méthyl N-vinylacétamide ;
- la N-vinylformamide ;
- la N-méthyl N-vinylformamide ;
- les N-vinyllactames comportant un groupe alkyle cyclique ayant de 4 à 9 atomes de carbone, tels que la N-vinylpyrrolidone, la N-butyrolactame et la N-vinylcaprolactame ;
- l'anhydride maléique ;
- l'acide itaconique ;
- l'alcool vinylique de formule $CH_2$=CHOH ;
- le chlorure de diméthyldiallyl ammonium ;
- le méthacrylate de diméthylaminoéthyl quaternisé (MADAME) ;
- le chlorure de (méth)acrylamido propyl triméthyl ammonium (APTAC et MAPTAC) ;
- le chlorure de méthylvinylimidazolium ;
- la 2-vinylpyridine ;

- la 4-vinylpyridine ;
- le (méth)acrylate de glycidyle ;
- les monomères vinyliques de formule (III) suivante :

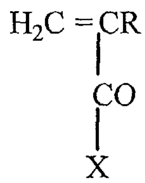

(III)

dans laquelle :

- R est choisi parmi H, $-CH_3$, $-C_2H_5$ ou $-C_3H_7$ ;
- X est choisi parmi :

- les oxydes d'alkyle de type -OR' où R' est un radical hydrocarboné, linéaire ou ramifié, saturé ou insaturé, ayant de 1 à 6 carbones, éventuellement substitué par au moins un atome d'halogène (iode, brome, chlore, fluor); un groupement sulfonique ($-SO_3^-$), sulfate ($-SO_4^-$), phosphate ($-PO_4H_2$); hydroxy (-OH); amine primaire ($-NH_2$); amine secondaire ($-NHR_1$), tertiaire ($-NR_1R_2$) ou quaternaire ($-N^+R_1R_2R_3$) avec $R_1$, $R_2$ et $R_3$ étant, indépendamment l'un de l'autre, un radical hydrocarboné, linéaire ou ramifié, saturé ou insaturé ayant 1 à 6 atomes de carbone, sous réserve que la somme des atomes de carbone de R' + $R_1$ + $R_2$ + $R_3$ ne dépasse pas 6 ; et
- les groupements $-NH_2$, $-NHR_4$ et $-NR_4R_5$ dans lesquels $R_4$ et $R_5$ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de $R_4$ + $R_5$ ne dépasse pas 6, lesdits $R_4$ et $R_5$ étant éventuellement substitués par un atome d'halogène (iode, brome, chlore, fluor) ; un groupement hydroxy (-OH); sulfonique ($-SO_3^-$); sulfate ($-SO_4^-$); phosphate ($-PO_4H_2$); amine primaire ($-NH_2$); amine secondaire ($-NHR_1$), tertiaire ($-NR_1R_2$) et/ou quaternaire ($-N^+R_1R_2R_3$) avec $R_1$, $R_2$ et $R_3$ étant, indépendamment l'un de l'autre, un radical hydrocarboné, linéaire ou ramifié, saturé ou insaturé ayant 1 à 6 atomes de carbone, sous réserve que la somme des atomes de carbone de $R_4$ + $R_5$ + $R_1$ + $R_2$ + $R_3$ ne dépasse pas 6 ; tels que la N,N diméthylacrylamide, et la N,N diéthylacrylamide.

Le ou les monomères hydrophobes (Ib), éventuellement présent en une quantité minoritaire pour former le squelette, sont choisis parmi les monomères suivants ou les sels de ceux-ci :

- l'acétate de vinyle de formule $CH_2=CH-OCOCH_3$ ;
- l'acrylonitrile ;
- le chlorure de vinyle ;
- le chlorure de vinylidène ;
- les monomères vinyliques de formule (III) suivante :

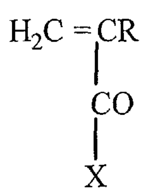

(III)

dans laquelle :

- R est choisi parmi H, $-CH_3$, $-C_2H_5$ ou $-C_3H_7$ ;
- X est choisi parmi :

- les oxydes d'alkyle de type -OR' où R' est un radical hydrocarboné, linéaire ou ramifié, saturé ou insaturé, ayant

de 7 à 22 carbones, éventuellement substitué par au moins un atome d'halogène (iode, brome, chlore, fluor); un groupement sulfonique ($-SO_3^-$), sulfate ($-SO_4^-$), phosphate ($-PO_4H_2$); hydroxy (-OH); amine primaire ($-NH_2$); amine secondaire ($-NHR_1$), tertiaire ($-NR_1R_2$) ou quaternaire ($-N^+R_1R_2R_3$) avec $R_1$, $R_2$ et $R_3$ étant, indépendamment l'un de l'autre, un radical hydrocarboné, linéaire ou ramifié, saturé ou insaturé ayant 7 à 22 atomes de carbone, sous réserve que la somme des atomes de carbone de R' + $R_1$ + $R_2$ + $R_3$ ne dépasse pas 22 ; et

- les groupements $-NH_2$, $-NHR_4$ et $-NR_4R_5$ dans lesquels $R_4$ et $R_5$ sont indépendamment l'un de l'autre des radicaux hydrocarbonés, linéaires ou ramifiés, saturés ou insaturés ayant 7 à 22 atomes de carbone, sous réserve que le nombre total d'atomes de carbone de $R_4$ + $R_5$ ne dépasse pas 22, lesdits $R_4$ et $R_5$ étant éventuellement substitués par un atome d'halogène (iode, brome, chlore, fluor); un groupement hydroxy (-OH); sulfonique ($-SO_3^-$); sulfate ($-SO_4^-$); phosphate ($-PO_4H_2$); aminé primaire ($-NH_2$); amine secondaire ($-NHR_1$), tertiaire ($-NR_1R_2$) et/ou quaternaire ($-N^+R_1R_2R_3$) avec $R_1$, $R_2$ et $R_3$ étant, indépendamment l'un de l'autre, un radical hydrocarboné, linéaire ou ramifié, saturé ou insaturé ayant 7 à 22 atomes de carbone, sous réserve que la somme des atomes de carbone de $R_4$ + $R_5$ + $R_1$ + $R_2$ + $R_3$ ne dépasse pas 22 ; tels que la N,N diméthylacrylamide, et la N,N diéthylacrylamide.

Ces éventuels monomères hydrophobes (Ib) sont généralement présents en une quantité suffisamment faible pour que le squelette hydrosoluble du polymère soit, précisément, soluble dans l'eau.

Les squelettes hydrosolubles, de manière préférentielle, sont neutralisés totalement ou partiellement par une base minérale ou organique.

Cette base peut être choisie, par exemple, parmi les sels de sodium, ammonium, lithium, calcium, magnésium, ammonium substitué par 1 à 4 groupes alkyle comportant de 1 à 15 atomes de carbone, ou encore parmi la mono-, la di-, et la triéthanolamine, l'aminométhylpropanediol, la N-méthyl-glucamine, et les acides aminés basiques, tels que l'arginine et la lysine, et leurs mélanges.

Le polymère, et en particulier le squelette hydrosoluble, peut être réticulé totalement ou partiellement, en utilisant par exemple les agents de réticulation ou composés à polyinsaturation oléfinique utilisés couramment pour la réticulation des polymères obtenus par polymérisation radicalaire. On peut citer comme exemples de ces agents de réticulation, le divinylbenzène, l'éther diallylique, la triallylamine, la tétraallyléthylènediamine, le dipropylèneglycol-diallyléther, les polyglycol-diallyléthers, l'hydroquinone-diallyl-éther, le triméthylolpropane-diallyléther, le tétraallyloxyéthane, les éthers allyliques d'alcools de la série des sucres, les polyallylesters, le tétrallyl-oxéthanoyle ou d'autres allyl- ou vinyléthers alcools polyfonctionnels, le triéthylèneglycol-divinyléther, les esters allyliques de l'acide vinylphosphonique et de l'acide phosphorique, les composés comportant deux ou trois groupes (méth)acrylates ou (méth)acrylamides tels que le diacrylate d'éthylèneglycol, le diacrylate de tétraéthylèneglycol, le diacrylate de butanediol, le méthacrylate d'allyle, le triméthylol propane triacrylate (TMPTA), le méthylène-bis-acrylamide ou leurs mélanges.

On utilisera plus particulièrement le méthylène-bis-acrylamide, le méthacrylate d'allyle ou le triméthylol propane triacrylate (TMPTA).

Le taux de réticulation varie, de préférence, de 0,01 à 10 % en moles, et plus particulièrement de 0,2 à 7 % en moles par rapport au polymère.

La réticulation du polymère, et en particulier du squelette hydrosoluble, peut également être effectuée en utilisant un macromonomère à LCST (II) multifonctionnel, par exemple porteur de deux ou trois fonctions insaturées, qui est introduit dans le milieu réactionnel lors de la polymérisation (en plus des composants a) et b)), dans des proportions adéquates, afin d'obtenir un taux de réticulation molaire de 0,01 à 10 %.

De préférence, le squelette hydrosoluble a une masse molaire allant de 1 000 g/mole à 50 000 000 g/mole, de préférence de 10 000 g/mole à 10 000 000 g/mole.

Selon l'invention, le polymère est obtenu par polymérisation radicalaire des monomères (Ia) et, éventuellement, (Ib) décrits plus haut et d'un ou plusieurs macromonomères répondant à la formule (II) déjà donnée ci-dessus.

Dans la formule (II), X est un groupe divalent choisi, par exemple, parmi -O-, -S-, -PH-, -NH-, et -NR°-, où R° est un groupe alkyle de 1 à 6 atomes de carbone.

A est un groupe comprenant au moins une liaison hydrocarbonée insaturée susceptible d'être polymérisée (par voie radicalaire).

Le groupe A peut être choisi, par exemple, parmi les groupes vinyle, allyle, acryle et méthacryle.

Le macromonomère (II) comprend également, de manière fondamentale, des unités à LCST B.

On peut définir les unités à LCST des polymères utilisés dans l'invention, comme étant des unités dont la solubilité dans l'eau est modifiée au-delà d'une certaine température. Il s'agit d'unités présentant une température de démixtion par chauffage (ou point de trouble) définissant leur zone de solubilité dans l'eau. La température de démixtion minimale obtenue en fonction de la concentration en polymère est appelée "LCST" (Lower Critical Solution Température). Pour chaque concentration en polymère, une température de démixtion par chauffage est observée ; elle est supérieure à la LCST qui est le point minimum de la courbe. En dessous de cette température, le polymère constituant l'unité à LCST est soluble dans l'eau ; au-dessus de cette température, le polymère constituant l'unité à LCST perd sa solubilité dans l'eau.

Certains de ces polymères à LCST sont notamment décrits dans les articles de TAYLOR et al., Journal of Polymer Science, part A : Polymer Chemistry, 1975, 13, 2 551 [11] ; de J. BAILEY et al., Journal of Applied Polymer Science, 1959, 1,56 [12] ; et de HESKINS et al., Journal of Macromolecular Science, Chemistry A2, 1968, vol. 8, 1 441 [13].

Par soluble dans l'eau à la température T, on entend que les unités présentent une solubilité à T, d'au moins 1 g/l, de préférence d'au moins 2 g/l.

La mesure de la LCST peut se faire visuellement : on détermine la température à laquelle apparaît le point de trouble de la solution aqueuse ; ce point de trouble se traduit par l'opacification de la solution, ou perte de transparence.

D'une manière générale, une composition transparente aura une valeur de transmittance maximum de la lumière, quelle que soit la longueur d'onde comprise entre 400 et 800 nm, à travers un échantillon de 1 cm d'épaisseur, d'au moins 85 %, de préférence d'au moins 90 %.

La transmittance peut être mesurée en plaçant un échantillon de 1 cm d'épaisseur dans le rayon lumineux d'un spectrophotomètre travaillant dans les longueurs d'onde du spectre lumineux.

Les unités à LCST des polymères utilisés dans l'invention peuvent être constituées par un ou plusieurs polymères choisis parmi les polymères suivants :

- les polyéthers tels que le poly oxyde d'éthylène (POE), le poly oxyde de propylène (POP), les copolymères statistiques d'oxyde d'éthylène (OE) et d'oxyde de propylène (OP) ;
- les polyvinylméthyléthers ;
- les dérivés polymériques et copolymériques N-substitués de l'acrylamide ayant une LCST, tels que le poly N-isopropylacrylamide (Nipam) et le poly N-éthylacrylamide ; et
- le poly-N-vinylcaprolactame et les copolymères de N-vinyl caprolacatame.

De préférence, les unités à LCST sont constituées de poly oxyde de propylène $(POP)_n$ où n est un nombre entier de 10 à 50, ou de copolymères statistiques d'oxyde d'éthylène (OE) et d'oxyde de propylène (OP), représentés par la formule $(OE)_m$ $(OP)_n$, dans laquelle m est un nombre entier allant de 1 à 40, de préférence de 2 à 20, de préférence encore de 2 à 10, et n est un nombre entier allant de 10 à 60, de préférence de 20 à 50.

De préférence, la masse molaire de ces unités à LCST est de 500 à 5 300 g/mole, de préférence encore de 1 000 à 4 000 g/mole, et mieux de 1 500 à 3 000 g/mole.

On a constaté que la répartition statistique des motifs OE et OP se traduit par l'existence d'une température inférieure critique de démixtion, au-delà de laquelle une séparation de phases macroscopique est observée. Ce comportement est différent de celui des copolymères (OE) (OP) à blocs, qui micellisent au-delà d'une température critique dite de micellisation (agrégation à l'échelle microscopique).

Les unités à LCST peuvent donc notamment être des copolymères statistiques d'oxyde d'éthylène et d'oxyde de propylène, aminés, notamment monoaminés, diaminés ou triaminés.

Parmi ces polymères à LCST, commercialement disponibles, on peut citer les copolymères vendus sous la dénomination Jeffamine® par HUNTSMAN, et notamment la Jeffamine® XTJ-507 (M-2005), la Jeffamine® D-2000 et la Jeffamine® XTJ-509 (ou T-3000).

Les unités à LCST peuvent également être issues de copolymères OE/OP statistiques à extrémités OH, tels que ceux vendus sous la dénomination Polyglycols® P41 et B11 par Clariant.

On peut aussi utiliser dans l'invention comme unités à LCST, les dérivés polymériques et copolymériques N-substitués de l'acrylamide ayant une LCST, ainsi que le poly-N-vinyl caprolactame et les copolymères de N-vinylcaprolactame.

A titre d'exemples de dérivés polymériques et copolymériques N-substitués de l'acrylamide ayant une LCST, on peut citer le poly N-isopropylacrylamide, le poly N-éthylacrylamide et les copolymères de N-isopropylacrylamide (ou de N-éthylacrylamide) et d'un ou plusieurs monomères choisis parmi les monomères (Ia) et (Ib).

La masse molaire de ces polymères est de préférence de 1 000 g/mole à 30 000 g/mole.

La synthèse de ces polymères peut être réalisée par polymérisation radicalaire à l'aide d'un couple d'amorceurs tel que le chlorhydrate d'aminoéthanethiol, en présence de persulfate de potassium, afin d'obtenir des oligomères précurseurs ayant une extrémité réactive aminée.

A titre d'exemples de copolymères de N-vinylcaprolactame, on peut citer les copolymères de N-vinyl caprolactame et d'un ou plusieurs monomère(s) choisi(s) parmi les monomères vinyliques (Ia) et (Ib), définis plus haut.

La masse molaire de ces polymères ou copolymères de N-vinylcaprolactame est généralement de 1 000 g/mole à 30 000 g/mole.

La synthèse de ces composés peut être réalisée par polymérisation radicalaire à l'aide d'un couple d'amorceurs tel que le chlorhydrate d'aminoéthanethiol, en présence de persulfate de potassium, afin d'obtenir des unités à LCST ayant une extrémité réactive aminée.

La proportion massique des unités à LCST dans le polymère final est de préférence de 5 % à 70 %, notamment de 20 % à 65 %, et particulièrement de 30 % à 60 % en poids, par rapport au polymère final.

On a vu plus haut que la température de démixtion par chauffage desdites unités à LCST du polymère utilisé dans l'invention est de 5 à 40°C, de préférence de 10 à 35°C, de préférence encore de 10 à 30°C pour une concentration massique dans l'eau, de 1 % en poids desdites unités à LCST.

Un macromonomère à LCST (II) est obtenu par réaction entre une unité à LCST portant au moins un site réactif et un composé comportant au moins un site réactif complémentaire et au moins une liaison hydrocarbonée insaturée telle qu'une fonction vinyle ou allyle ; citons comme exemple l'acide (méth)acrylique, l'anhydride maléique, l'acide itaconique, l'alcool vinylique, l'acétate de vinyle, le (méth)acrylate de glycidyle, le 3-chloropropène, le 4-isocyanatostyrène et le chlorométhylstyrène. Un site réactif peut être notamment choisi parmi les fonctions alcool, ester de glycéryle, isocyanate, amine primaire, secondaire ou tertiaire, acide carboxylique ou halogène. Notamment, un site réactif du type acide carboxylique et ester va généralement réagir avec un site réactif du type alcool ou amine ; un site isocyanate va plutôt réagir avec un site alcool, et un site halogène avec un site alcool ou amine. Les réactions mises en jeu peuvent être par exemple une estérification, une transestérification, une amidification, ou encore une substitution nucléophile.

Selon l'invention, les polymères décrits plus haut sont préparés en réalisant la copolymérisation radicalaire par précipitation d'un ou plusieurs monomères hydrosolubles (Ia), déjà définis plus haut, éventuellement de un ou plusieurs monomères hydrophobes (Ib), déjà définis plus haut, en une quantité minoritaire par rapport auxdits monomères hydrosolubles (Ia), et d'un ou plusieurs macromonomères de formule (II), déjà décrits plus haut, dans un milieu comprenant un alcool et de l'eau (milieu hydroalcoolique), et en isolant le polymère obtenu.

L'alcool utilisé est miscible à l'eau et est généralement un alcool aliphatique comportant de 1 à 4 atomes de carbone, comme par exemple le méthanol, l'éthanol, le propanol, l'isopropanol et de préférence le tertiobutanol. La teneur massique en eau est généralement inférieure ou égale à 10%, de préférence inférieure à 5% en poids.

De tels alcools, selon une caractéristique avantageuse du procédé, sont totalement compatibles d'un point de vue cosmétique.

Les monomères et les macromonomères sont dissous totalement ou partiellement dans le milieu de polymérisation, tandis que le polymère y est insoluble.

La réaction de polymérisation s'effectue à une température comprise entre -10°C et 100°C, de préférence entre 20°C et 70°C.

De manière classique, la copolymérisation est réalisée en présence d'un initiateur de polymérisation.

Les initiateurs de polymérisation sont des composés qui génèrent des radicaux libres. Ces initiateurs peuvent être choisis, par exemple, parmi l'eau oxygénée ; les composés organiques peroxydés tels que le peroxyde de benzène, l'hydroperoxyde de tertiobutyle, l'hydroperoxyde de méthyléthylcétone ; les composés diazotés tels que l'azobisisobutyronitrile, l'azobisdiméthylvaleronitrile ; les couples oxydant/réducteur tels que peroxodisulfate d'ammonium/métabisulfite de sodium, et peroxodisulfate d'ammonium/N,N,N',N' tétraméthylène diamine.

La réaction de polymérisation peut également être initiée à l'aide de photoinitiateurs ou par une radiation telle que des UV, des neutrons ou par plasma.

La polymérisation s'effectue en milieu inerte, de préférence sous atmosphère d'azote ou d'argon.

Le polymère apparaît dans le milieu réactionnel sous la forme d'un précipité blanc. Il peut être isolé facilement en utilisant les procédés usuels de séparation, d'évaporation et de séchage. Par exemple, le tertiobutanol peut être éliminé par filtration ou distillation.

Les polymères obtenus répondent aux propriétés d'application décrites dans les demandes de brevets FR-01 00485, FR-01 00480, FR-01 00478, FR-01 00481, FR-01 00483, FR-01 05112.

L'invention concerne également une composition aqueuse comprenant au moins un polymère, tel que défini plus haut, et une phase aqueuse.

Le ou les polymères selon l'invention sont de préférence présent(s) dans les compositions aqueuses en une quantité de préférence comprise entre 0,01 et 20 % en poids, notamment de 0,05 à 15 % en poids, et en particulier de 0,1 à 10 % en poids.

Ces compositions et les polymères qu'elles comprennent trouvent une application toute particulière dans les domaines cosmétique et dermatologique.

Ladite composition comprend, outre le polymère tel que ci-dessus défini, une phase aqueuse, qui peut comprendre, outre de l'eau, une eau florale telle que l'eau de bleuet, une eau minérale telle que l'eau de VITTEL, l'eau de LUCAS ou l'eau de LA ROCHE POSAY et/ou une eau thermale.

Il est possible d'ajouter dans ladite composition aqueuse les constituants usuellement utilisés dans le type d'application envisagé. Bien entendu l'homme du métier veillera à choisir ces éventuels constituants complémentaires, et/ou leur quantité, de manière telle que les propriétés avantageuses de la composition selon l'invention ne soient pas, ou substantiellement pas, altérées par l'adjonction envisagée.

Ladite composition aqueuse peut former tout ou partie d'une composition cosmétique ou dermatologique qui

peut donc comprendre par ailleurs un milieu cosmétiquement ou dermatologiquement acceptable, c'est-à-dire un milieu compatible avec une application sur les matières kératiniques telles que la peau, les ongles, les cheveux, les cils et sourcils, les muqueuses et les semi-muqueuses, et toute autre zone cutanée du corps et du visage.

La composition de l'invention peut se présenter sous toutes les formes galéniques habituellement utilisées dans les domaines cosmétique et dermatologique. Elle peut se présenter par exemple sous forme de gels, sous forme d'émulsions huile-dans-eau (H/E) ou eau-dans-huile (E/H) ou d'émulsion multiple. On entend ici par "émulsion" aussi bien des dispersions sans émulsionnants que des dispersions comportant des émulsionnants ou encore des dispersions stabilisées par des particules solides ou par des sphérules lipidiques de type ionique ou non ionique.

Dans les compositions de l'invention sous forme d'émulsion, la phase aqueuse de la composition peut être présente en une concentration allant par exemple de 5 à 80 % et de préférence 30 à 70 % en poids par rapport au poids total de la composition, et la phase huileuse peut être présente en une concentration allant de 5 à 70 % et de préférence de 10 à 50 % en poids par rapport au poids total de la composition.

La phase grasse ou phase huileuse contient habituellement au moins une huile. Comme huiles utilisables dans la composition de l'invention, on peut citer par exemple :

- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules $R^1COOR^2$ et $R^1OR^2$ dans laquelle $R^1$ représente le reste d'un acide gras comportant de 8 à 29 atomes de carbone, et $R^2$ représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les alcools gras alcoylés et notamment éthoxylés tels que l'oleth-12 ;
- les huiles fluorées partiellement hydrocarbonées et/ou siliconées comme celles décrites dans le document JP-A-2-295912. Comme huiles fluorées, on peut citer aussi le perfluorométhylcyclopentane et le perfluoro-1,3 diméthylcyclohexane, vendus sous les dénominations de "FLUTEC PC1®" et "FLUTEC PC3®" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem ; le nonafluorométhoxybutane vendu sous la dénomination "MSX 4518®" par la Société 3M et le nonafluoroéthoxyisobutane ; les dérivés de perfluoromorpholine, tels que la 4-trifluorométhyl perfluoromorpholine vendue sous la dénomination "PF 5052®" par la Société 3M ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphénylsiloxanes, les diphényldiméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les gommes telles que les gommes de silicone (diméthiconol) ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Les émulsions peuvent contenir au moins un émulsionnant choisi parmi les émulsionnants amphotères, anioniques, cationiques ou non ioniques, utilisés seuls ou en mélange.

Les émulsionnants sont choisis de manière appropriée suivant l'émulsion à obtenir (E/H ou H/E).

Pour les émulsions H/E, on peut citer par exemple les émulsionnants suivants :

- comme émulsionnants amphotères, les N-acylaminoacides tels que les N-alkyl-aminoacétates et le cocoamphodiacetate disodique et les oxydes d'amines tels que l'oxyde de stéaramine ;
- comme émulsionnants anioniques, les acylglutamates tels que le "disodium hydrogenated tallow glutamate" (AMISOFT HS-21[R] commercialisé par la société Ajinomoto) ; les acides carboxyliques et leurs sels tels que le stéarate de sodium ; les esters phosphoriques et leurs sels tels que le "DEA oleth-10 phosphate" ; les sulfosuccinates tels que le "Disodium PEG-5 citrate lauryl sulfosuccinate" et le "Disodium ricinoleamido MEA sulfosuccinate" ;
- comme émulsionnants cationiques, les alkylimidazolidinium tels que l'étho-sulfate d'isostéaryléthylimidonium ; les sels d'ammonium tels que le chlorure de N,N,N-triméthyl-1-docosanaminium (Behentrimonium chloride) ;
- comme émulsionnants non ioniques, les esters et éthers de sucres tels que le stéarate de sucrose, le cocoate de sucrose, et le mélange de stéarate de sorbitan et de cocoate de sucrose commercialisé par la société ICI sous la dénomination d'Arlatone 2121[R] ; les esters de polyol, notamment de glycérol ou de sorbitol, tels que le stéarate de glycéryle, le stéarate de polyglycéryl-2, le stéarate de sorbitan ; les éthers de glycérol ; les éthers oxyéthylénés et/ou oxypropylénés tels que l'éther oxyéthyléné, oxypropyléné de l'alcool laurique à 25 groupes oxyéthylénés et 25 groupes oxypropylénés (nom CTFA "PPG-25 laureth-25") et l'éther oxyéthylèné du mélange d'alcools gras en $C_{12}$-$C_{15}$ comportant 7 groupes oxyéthylénés (nom CTFA "C12-15 Pareth-7") ; les polymères d'éthylène glycol, tels que le PEG-100.

Pour les émulsions E/H, on peut citer par exemple comme émulsionnants les esters gras de polyol, notamment de glycérol ou de sorbitol, et notamment les esters isostéariques, oléiques et ricinoléiques de polyol, tels que le mélange de petrolatum, d'oléate de polyglycéryl-3, d'isostéarate de glycéryle, l'huile de ricin hydrogénée et d'ozokérite, vendu sous la dénomination PROTEGIN W[R] par la société Goldschmidt, l'isostéarate de sorbitan, le di-isostéarate de polyglycéryle, le sesquiisostéarate de polyglycéryle-2 ; les esters et éthers de sucres tels que le "Methyl glucose dioleate" ; les sels d'acide gras tels que le lanolate de magnésium ; les dimethicone copolyols et alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination Abil EM 90[R] par la société Goldschmidt.

Les émulsionnants peuvent être introduits tels quels ou sous forme de mélange avec d'autres émulsionnants et/ou avec d'autres composés tels que des alcools gras ou des huiles.

La composition de l'invention peut contenir en plus, des adjuvants classiques tels que des colorants hydrosolubles ou liposolubles, des pigments, des parfums, des conservateurs, des filtres solaires, des séquestrants (EDTA), des actifs liposolubles ou hydrosolubles, des ajusteurs de pH (acides ou bases). Ces adjuvants sont utilisés dans les proportions habituelles dans le domaine cosmétique, et par exemple de 0,01 à 20 % en poids par rapport au poids total de la composition. Ces adjuvants ainsi que leurs concentrations doivent être tels qu'ils ne modifient pas la propriété recherchée pour la composition.

Comme actifs utilisables dans la composition de l'invention, on peut citer par exemple les agents hydratants et par exemple les hydrolysats de protéines et les polyols tels que la glycérine, les glycols comme les polyéthylène glycols, et les dérivés de sucre ; les extraits naturels ; les oligomères procyannidoliques ; les vitamines comme la vitamine A (rétinol), la vitamine C (acide ascorbique), la vitamine E (tocophérol), la vitamine B5 (panthénol), la vitamine B3 ou PP (niacinamide) ; l'urée ; la caféine ; les dépigmentants tels que l'acide kojique et l'acide caféique ; l'acide salicylique ; les alpha-hydroxyacides tels que l'acide lactique et l'acide glycolique ; les rétinoïdes tels que les caroténoïdes ; l'hydrocortisone ; la mélatonine ; les extraits d'algues, de champignons, de végétaux, de levures, de bactéries ; les protéines hydrolysées, partiellement hydrolysées ou non hydrolysées, les enzymes ; les actifs antibactériens pour le traitement des peaux grasses comme le 2,4,4'-trichloro-2'-hydroxy diphényl éther (ou triclosan), le

3,4,4'-trichlorocarbanilide (ou triclocarban) ; les fibres ; et leurs mélanges.

Le ou les actifs peuvent être par exemple présents en une concentration allant généralement de 0,01 à 20 %, de préférence de 0,1 à 10 % et mieux de 0,5 à 5 % du poids total de la composition.

Les filtre U.V. (ou filtre solaire) peuvent être choisis parmi les filtres chimiques, les filtres physiques et les mélanges de tels filtres. Comme exemples de filtres U.V., on peut citer :

- le butylméthoxydibenzoylméthane vendu notamment par la société Hoffmann-Laroche sous la dénomination Parsol 1789 ;
- l'octocrylène vendu notamment par la société BASF sous la dénomination Uvinul N539 ;
- l'octylsalicylate vendu notament par la société Haarman-Reimer sous la dénomination Neo Heliopan OS ;
- l'octylméthoxycinnamate vendu notamment par la société Hoffmann-Laroche sous la dénomination Parsol MCX ;
- l'acide phénylbenzimidazole sulfonique vendu notamment par la société Merck sous la dénomination Eusolex 232 ;
- les oxybenzones telles que les benzophénones-3, -4 ou -5 ;
- les silicones benzotriazoles et en particulier le drométrizole trisiloxane ;
- l'acide téréphtalylidène di-camphre sulfonique ; et
- les oxydes de titane ou de zinc, sous forme de micro- ou nanoparticules (nanopigments) éventuellement enrobées.

La composition de l'invention peut contenir en outre des charges en vue de modifier la texture de la composition. Comme charges qui peuvent être utilisées dans la composition de l'invention, on peut citer par exemple, outre les pigments, la poudre de silice ; le talc ; les particules de polyamide et notamment celles vendues sous la dénomination ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinique, commercialisées sous la dénomination DRY-FLO par la société National Starch ; les microbilles de résine de silicone telles que celles commercialisées sous la dénomination TOSPEARL par la société Toshiba Silicone ; et leurs mélanges. Ces charges peuvent être présentes dans des quantités allant de 0 à 40% en poids et de préférence de 1 à 10 % en poids par rapport au poids total de la composition.

Ladite composition trouve donc une application particulière comme composition cosmétique, susceptible d'être appliquée sur les matières kératiniques (la peau, les ongles, les cheveux, les cils et sourcils, les muqueuses et les semi-muqueuses, et toute autre zone cutanée du corps et du visage).

La composition selon l'invention contient un milieu physiologiquement acceptable et trouve son application dans un grand nombre de traitements notamment cosmétiques des matières kéartiniques en particulier pour le soin (par exemple anti-âge), le nettoyage (et démaquillage), le maquillage et/ou la protection solaire des matières kératiniques.

Aussi, la présente invention a pour objet l'utilisation cosmétique de la composition telle que définie ci-dessus, pour le soin et/ou le nettoyage et/ou le maquillage et/ou la protection solaire des matières kératiniques.

L'invention a encore pour objet un procédé de traitement cosmétique pour le soin et/ou le nettoyage et/ou le maquillage et/ou la protection solaire des matières kératiniques, consistant à appliquer sur les matières kératiniques, une composition telle que définie ci-dessus.

L'invention va maintenant être décrite en référence aux exemples suivants, relatifs à la préparation de macromonomères entrant dans la composition des polymères de l'invention, à la préparation de polymères de l'invention et à des compositions cosmétiques comprenant les polymères de l'invention.

## Exemples

### Exemple 1

Synthèse de macromonomères à LCST

### Exemple 1.1

Synthèse d'un macromonomère à LCST portant des chaînes d'un copolymère d'oxypropylène et d'oxyéthylène statistique (Jeffamine® M-2005).

Dans un réacteur de 500 ml sont introduits 100 grammes de Jeffamine® M-2005 (Hunstman), 4,3 grammes d'acide acrylique et 2,6 grammes de carbonate de potassium. Le milieu réactionnel est laissé sous agitation pendant

2 heures à reflux. L'acide chlorhydrique formé est éliminé sous vide. Le macromonomère obtenu est constitué d'un oligomère statistique de polyoxypropylène (39 moles) et de polyoxyéthylène (6 moles) portant à l'une de ses extrémités une fonction méthylacrylamide.

Exemple 1.2

Synthèse d'un macromonomère à LCST difonctionnel, portant des chaînes polyoxypropylène (Jeffamine® D-2000).

Dans un réacteur de 500 ml sont introduits 100 grammes de Jeffamine® D-2000 (Hunstman), 5,2 grammes d'acide méthacrylique et 2,6 grammes de carbonate de potassium. Le milieu réactionnel est laissé sous agitation pendant 2 heures à reflux. L'acide chlorhydrique formé est éliminé sous vide. Le macromonomère obtenu est constitué d'un oligomère de polyoxypropylène portant à ses deux extrémités une fonction méthylacrylamide.

Exemple 1.2

Synthèse d'un macromonomère portant des chaînes poly N-isopropylacrylamide.

Cette synthèse comporte deux étapes :

- Synthèse d'oligomères pNIPAM portant une extrémité réactive aminée.

  Dans un ballon tricol de 2500 ml muni d'un réfrigérant et d'une arrivée d'azote sont introduits 80 grammes de N-isopropylacrylamide et 800 ml de d'eau osmosée. Ce mélange est chauffé sous agitation à 29°C à l'aide d'un bain-marie et placé sous un barbotage d'azote. Après 45 minutes, 1,61 grammes de chlorhydrate d'aminoéthanethiol préalablement dissous dans 40 ml d'eau osmosée sont ajoutés au milieu réactionnel. 5 minutes après, 1,91 grammes de persulfate de potassium dissous dans 80 ml d'eau osmosée sont ajoutés au milieu réactionnel. Ce milieu réactionnel est maintenu sous agitation et atmosphère d'azote pendant 3 heures à 29°C.

  Les oligomères de poly N-isopropylacrylamide (pNIPAM) monoaminés ainsi synthétisés sont isolés par chauffage du milieu réactionnel à 45°C et filtration.

- Synthèse d'un macromonomère pNIPAM portant une extrémité acrylamide.

Dans un réacteur de 500 ml sont introduits 80 grammes d'oligomères pNIPAM portant une extrémité aminée, 1,1 grammes d'acide méthacrylique et 0,6 grammes de carbonate de potassium. Le milieu réactionnel est laissé sous agitation pendant 2 heures à reflux. L'acide chlorhydrique formé est éliminé sous vide. Le macromonomère obtenu est constitué d'un oligomère de pNIPAM portant à l'une de ses extrémités une fonction méthyl-acrylamide.

Exemple 2

Copolymérisation de(s) monomère(s) ayant une liaison hydrocarbonée insaturée et d'un macromonomère à LCST.

300 grammes de tertio-butanol sont introduits dans un réacteur de 2 litres muni d'un agitateur, d'un réfrigérant, d'un thermomètre, d'un bain thermostaté, d'une ampoule d'introduction de réactifs et d'une arrivée de gaz afin de contrôler l'atmosphère au-dessus du milieu réactionnel. Le milieu réactionnel est placé sous atmosphère d'azote, après un bullage d'azote pendant 30 minutes. X grammes d'acide acrylamido-2-méthylpropane sulfonique (AMPS) sont introduits sous agitation et filet d'azote; le pH du milieu réactionnel est alors égal à 1. De l'ammoniac gazeux est introduit au-dessus du milieu réactionnel jusqu'à ce que son pH soit compris entre 7 et 8. Si nécessaire, $Y_i$ grammes des autres comonomères i comportant au moins une fonction insaturée et Z grammes de(s) macromonomère(s) à LCST, préparés dans l'exemple 1, sont introduits dans le milieu réactionnel qui est alors maintenu sous agitation pendant 1 heure ; le pH est mesuré de façon continue afin de vérifier que sa valeur se situe entre 7 et 8. L'atmosphère au-dessus du milieu réactionnel est de nouveau saturée par de l'azote afin de diminuer la teneur en oxygène dans la phase liquide à une teneur inférieure à 1 ppm. 1 gramme d'azobisisobutyronitrile (AIBN) est introduit dans le milieu réactionnel sous azote et la température est ajustée à 60°C. Dès que la température atteint 60°C, la réaction de polymérisation débute. Après une trentaine de minutes, la température est ajustée à la température d'ébullition du tertio-butanol; le milieu réactionnel est alors maintenu au reflux sous agitation pendant 2 heures.

Le milieu réactionnel devient alors une suspension visqueuse de polymère dans le tertio-butanol; le polymère est récupéré par simple filtration du tertiobutanol, suivie d'un séchage sous vide.

Ce mode opératoire est utilisé pour synthétiser les polymères hydrosolubles suivants, comportant des unités monomères de type (la) et/ou (Ib) et des macromonomères à LCST (II).

Exemple 2.1

| Réactifs et Solvant | Masse (g) |
|---|---|
| Tertiobutanol | 300 |
| AMPS sous forme sel d'ammonium | 84 |
| Macromonomère dérivé de Jeffamine® M-2005 | 36 |
| AIBN | 1 |

Le copolymère obtenu est composé de 70% en masse d'AMPS (sel d'ammonium) et de 30% de macromonomère de Jeffamine® M-2005 ; cela correspond à un taux molaire de 3,7% de macromonomère dans le copolymère. Ce polymère est non réticulé.

Exemple 2.2

| Réactifs et Solvant | Masse (g) |
|---|---|
| Tertiobutanol | 300 |
| AMPS sous forme sel d'ammonium | 60 |
| Macromonomère dérivé de Jeffamine® M-2005 | 60 |
| AIBN | 1 |

Le copolymère obtenu est composé de 50% en masse d'AMPS (sel d'ammonium) et de 50% de macromonomère de Jeffamine® M-2005 ; cela correspond à un taux molaire de 8,2% de macromonomère dans le copolymère. Ce polymère n'est pas réticulé.

Exemple 2.3

| Réactifs et Solvant | Masse (g) |
|---|---|
| Tertiobutanol | 300 |
| AMPS sous forme sel d'ammonium | 51 |
| N-vinylacétamide | 21 |
| Macromonomère dérivé de Jeffamine® M-2005 | 48 |
| AIBN | 1 |
| TMPTA | 1,51 |

Le copolymère obtenu est composé de 42 % en masse d'AMPS (sel d'ammonium), de 17,3 % de N-vinylacétamide, de 39,5 % de macromonomère de Jeffamine® M-2005 et de 1,2 % de TMPTA ; cela correspond à un taux molaire de 3,8 % de macromonomère dans le copolymère. Ce polymère est réticulé et le taux de réticulation molaire est égal à 1 %.

Exemple 2.4

| Réactifs et Solvant | Masse (g) |
|---|---|
| Tertiobutanol | 300 |
| AMPS sous forme sel d'ammonium | 84 |
| Macromonomère dérivé de Jeffamine® M-2005 | 36 |
| Macromonomère dérivé de Jeffamine® D-2000 | 3,9 |
| AIBN | 1 |

Le copolymère obtenu est composé de 67,8% en masse d'AMPS (sel d'ammonium) et de 29% de macromonomère de Jeffamine® M-2005 ; cela correspond à un taux molaire de macromonomères à LCST, égal à 3,7 %. Ce polymère est réticulé par le macromonomère difonctionnel issu de la Jeffamine® D-2000 et le taux de réticulation molaire est égale à 0,5%.

Exemple 2.5

| Réactifs et Solvant | Masse (g) |
|---|---|
| Tertiobutanol | 300 |
| AMPS sous forme sel d'ammonium | 60 |
| Macromonomère dérivé de pNIPAM | 60 |
| AIBN | 1 |

Le copolymère obtenu est composé de 50% en masse d'AMPS (sel d'ammonium) et de 50% de macromonomère de pNIPAM ; cela correspond à un taux molaire de 2,2% de macromonomère à LCST dans le copolymère. Ce polymère n'est pas réticulé.

Exemple 3

Pouvoir thermogélifiant des polymères synthétisés.

Le pouvoir thermogélifiant des copolymères précédents a été mis en évidence par des mesures rhéologiques (Rhéomètre Haake RS150, cône/plan 35mm/2°), à partir de solutions à 1% de polymère dans l'eau, soumises à une vitesse de cisaillement de $10s^{-1}$. Ces mesures permettent de déterminer la température au-delà de laquelle la viscosité augmente par chauffage, appelée température de gélification (Tgel).

| Polymère | Tgel (°C) |
|---|---|
| Exemple 2.1 | 32 |
| Exemple 2.2 | 27 |
| Exemple 2.3 | 29 |
| Exemple 2.4 | 33 |
| Exemple 2.5 | 34 |

Tous les polymères thermogélifiants obtenus ont une température de gélification dans l'eau, à une concentration de 1%, inférieure à 45°C, contrairement aux polymères décrits dans le brevet EP 1 069 142.

Dans les exemples suivants, on fournit des exemples de compositions cosmétiques comprenant le polymère de l'invention.

Exemple 4

Lait pour le corps.

Composition

| Phase aqueuse : | |
|---|---|
| Polymère de l'exemple 2.2 : | 0,4 g |
| Conservateur | 0,2 g |
| Eau déminéralisée | 84,4 g |

| Phase huileuse : | |
|---|---|
| Huile de Parléam | 9 g |
| Cyclohexadiméthylsiloxane | 6 g |

La phase aqueuse est préparée par dissolution du polymère de l'exemple 2.2 dans de l'eau déminéralisée contenant le conservateur, sous agitation pendant 2 heures. La phase huileuse est alors introduite lentement dans la phase aqueuse sous agitation à l'aide d'un mélangeur de type Moritz avec une vitesse de 4000 RPM pendant 20 minutes.

Le polymère de l'exemple 2.2 permet à lui seul d'émulsionner la totalité de la phase huile. La formule obtenue est une belle émulsion fluide pouvant être utilisée comme lait pour le corps.

Exemple 5

Composition moussante fluide.

Composition

| | |
|---|---|
| Polymère de l'exemple 2.5 : | 0,4 g |
| Glycérine | 5 g |
| Conservateur | 0,4 g |
| Eau déminéralisée | 94,2 g |

Cette composition moussante est obtenue après dissolution du polymère de l'exemple 2.5 sous forme de poudre dans de l'eau déminéralisée sous agitation à 25°C pendant 2 heures ; les autres constituants sont alors introduits dans cette solution qui est maintenue sous agitation pendant 30 minutes.

La formule obtenue est une composition moussante fluide pouvant être utilisée entre 5°C et 50°C. Le polymère de l'exemple 2.5 permet à lui seul d'obtenir un pouvoir moussant.

REFERENCES

[1] D. HOURDET et al., Polymer, 1994, vol. 35, n° 12, pages 2 624-2 630.

[2] F. L'ALLORET et al., Coll. Polym. Sci., 1995, vol. 273, n° 12, pages 1 163-1 173.

[3] F. L'ALLORET, Revue de l'Institut Français du Pétrole, 1997, vol. 52, n° 2, pages 117-128.

[4] EP-A-0 583 814.

[5] EP-A-0 629 649.

[6] WO-A-95 24430.

[7] US-A-5 939 485.

[8] WO-A-97 00275.

[9] WO-A-98 48768.

[10] WO-A-00 35961.

[11] Articles de TAYLOR et al., Journal of Polymer Science, part A : Polymer Chemistry, 1975, 13, 2 551.

[12] J. BAILEY et al., Journal of Applied Polymer Science, 1959, 1,56.

[13] HESKINS et al., Journal of Macromolecular Science, Chemistry A2, 1968, 1 441.

[14] D. HOURDET et al., Polymer, 1997, vol. 38, n° 10, pages 2 535 - 2 547.

[15] EP-A-1 069 142.

## Revendications

**1.** Polymère hydrosoluble comprenant un squelette hydrosoluble et des unités latérales présentant dans l'eau une température inférieure critique de démixtion LCST, ledit polymère étant susceptible d'être obtenu par polymérisation radicalaire par précipitation de :

a) un ou plusieurs monomères hydrosolubles (Ia), et, éventuellement, d'une quantité minoritaire par rapport au(x)dit(s) monomère(s) (Ia) de un ou plusieurs monomères hydrophobes (Ib), lesdits monomères (Ia) et (Ib) portant au moins une fonction insaturée susceptible d'être polymérisée pour former le squelette hydrosoluble ;
b) un ou plusieurs macromonomères répondant à la formule (II) suivante :

$$A\text{-}X\text{-}B \qquad (III),$$

dans laquelle A est un groupe comprenant au moins une liaison hydrocarbonée insaturée susceptible d'être polymérisée, X est un groupe choisi parmi -O-, -S-, -PH-, -NH-, et NR° où R° est un groupe alkyle de 1 à 6 atomes de carbone, et B est une unité à LCST dont la température de démixtion par chauffage en solution aqueuse est de 5 à 40°C pour une concentration massique dans l'eau de 1 % de ladite unité.

**2.** Polymère selon la revendication 1, dans lequel la température de démixtion par chauffage en solution aqueuse des unités à LCST du polymère est de 10 à 35°C, et de préférence de 10 à 30°C, pour une concentration massique dans l'eau de 1 % desdites unités.

**3.** Polymère selon l'une quelconque des revendications 1 et 2, dans lequel le ou les monomères hydrosolubles (Ia) sont choisis parmi les monomères suivants, ou les sels de ceux-ci :

- l'acide (méth)acrylique ;
- l'acide acrylamido-2-méthylpropane sulfonique (AMPS) ;
- l'acide styrène sulfonique ;
- l'acide vinylsulfonique ;
- l'acide (méth)allylsulfonique ;
- le (méth)acrylamide ;
- l'acide vinyl phosphonique ;
- la N-vinylacétamide ;
- la N-méthyl N-vinylacétamide ;
- la N-vinylformamide ;
- la N-méthyl N-vinylformamide ;
- les N-vinyllactames comportant un groupe alkyle cyclique ayant de 4 à 9 atomes de carbone, tels que la N-vinylpyrrolidone, la N-butyrolactame et la N-vinylcaprolactame ;
- l'anhydride maléique ;
- l'acide itaconique ;

- l'alcool vinylique de formule $CH_2$=CHOH ;
- le chlorure de diméthyldiallyl ammonium ;
- le méthacrylate de diméthylaminoéthyl quaternisé (MADAME) ;
- le chlorure de (méth)acrylamido propyl triméthyl ammonium (APTAC et MAPTAC) ;
- le chlorure de méthylvinylimidazolium ;
- la 2-vinylpyridine ;
- la 4-vinylpyridine ;
- le (méth)acrylate de glycidyle ;
- les monomères vinyliques de formule (III) suivante :

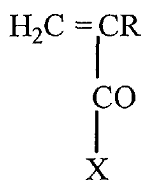

(III)

dans laquelle :

- R est choisi parmi H, $-CH_3$, $-C_2H_5$ ou $-C_3H_7$ ;
- X est choisi parmi :
    - les oxydes d'alkyle de type -OR' où R' est un radical hydrocarboné, linéaire ou ramifié, saturé ou insaturé, ayant de 1 à 6 carbones, éventuellement substitué par au moins un atome d'halogène (iode, brome, chlore, fluor); un groupement sulfonique ($-SO_3^-$), sulfate ($-SO_4^-$), phosphate ($-PO_4H_2$) ; hydroxy (-OH); amine primaire ($-NH_2$) ; amine secondaire ($-NHR_1$), tertiaire ($-NR_1R_2$) ou quaternaire ($-N^+R_1R_2R_3$) avec $R_1$, $R_2$ et $R_3$ étant, indépendamment l'un de l'autre, un radical hydrocarboné, linéaire ou ramifié, saturé ou insaturé ayant 1 à 6 atomes de carbone, sous réserve que la somme des atomes de carbone de R' + $R_1$ + $R_2$ + $R_3$ ne dépasse pas 6 ; et
    - les groupements $-NH_2$, $-NHR_4$ et $-NR_4R_5$ dans lesquels $R_4$ et $R_5$ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de $R_4$ + $R_5$ ne dépasse pas 6, lesdits $R_4$ et $R_5$ étant éventuellement substitués par un atome d'halogène (iode, brome, chlore, fluor) ; un groupement hydroxy (-OH); sulfonique ($-SO_3^-$); sulfate ($-SO_4^-$); phosphate ($-PO_4H_2$); amine primaire ($-NH_2$); amine secondaire ($-NHR_1$), tertiaire ($-NR_1R_2$) et/ou quaternaire ($-N^+R_1R_2R_3$) avec $R_1$, $R_2$ et $R_3$ étant, indépendamment l'un de l'autre, un radical hydrocarboné, linéaire ou ramifié, saturé ou insaturé ayant 1 à 6 atomes de carbone, sous réserve que la somme des atomes de carbone de $R_4$ + $R_5$ + $R_1$ + $R_2$ + $R_3$ ne dépasse pas 6 ; tels que la N,N diméthylacrylamide, et la N,N diéthylacrylamide ;

**4.** Polymère selon l'une quelconque des revendications 1 à 3, dans lequel le ou les monomères hydrophobes (Ib), éventuellement présent(s) en une quantité minoritaire pour former le squelette hydrosoluble, sont choisis parmi les monomères suivants ou les sels de ceux-ci :

- l'acétate de vinyle de formule $CH_2$=CH-$OCOCH_3$ ;
- l'acrylonitrile ;
- le chlorure de vinyle ;
- le chlorure de vinylidène ;
- les monomères vinyliques de formule (III) suivante :

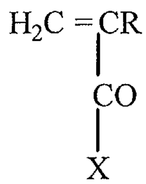

(III)

dans laquelle :

- R est choisi parmi H, $-CH_3$, $-C_2H_5$ ou $-C_3H_7$ ;
- X est choisi parmi :

  - les oxydes d'alkyle de type -OR' où R' est un radical hydrocarboné, linéaire ou ramifié, saturé ou insaturé, ayant de 7 à 22 carbones, éventuellement substitué par au moins un atome d'halogène (iode, brome, chlore, fluor); un groupement sulfonique ($-SO_3^-$), sulfate ($-SO_4^-$), phosphate ($-PO_4H_2$); hydroxy (-OH); amine primaire ($-NH_2$); amine secondaire ($-NHR_1$), tertiaire ($-NR_1R_2$) ou quaternaire ($-N^+R_1R_2R_3$) avec $R_1$, $R_2$ et $R_3$ étant, indépendamment l'un de l'autre, un radical hydrocarboné, linéaire ou ramifié, saturé ou insaturé ayant 7 à 22 atomes de carbone, sous réserve que la somme des atomes de carbone de R' + $R_1$ + $R_2$ + $R_3$ ne dépasse pas 22 ; et
  - les groupements $-NH_2$, $-NHR_4$ et $-NR_4R_5$ dans lesquels $R_4$ et $R_5$ sont indépendamment l'un de l'autre des radicaux hydrocarbonés, linéaires ou ramifiés, saturés ou insaturés ayant 7 à 22 atomes de carbone, sous réserve que le nombre total d'atomes de carbone de $R_4$ + $R_5$ ne dépasse pas 22, lesdits $R_4$ et $R_5$ étant éventuellement substitués par un atome d'halogène (iode, brome, chlore, fluor) ; un groupement hydroxy (-OH); sulfonique ($-SO_3^-$) ; sulfate ($-SO_4^-$) ; phosphate ($-PO_4H_2$); amine primaire ($-NH_2$); amine secondaire ($-NHR_1$), tertiaire ($-NR_1R_2$) et/ou quaternaire ($-N^+R_1R_2R_3$) avec $R_1$, $R_2$ et $R_3$ étant, indépendamment l'un de l'autre, un radical hydrocarboné, linéaire ou ramifié, saturé ou insaturé ayant 7 à 22 atomes de carbone, sous réserve que la somme des atomes de carbone de $R_4$ + $R_5$ + $R_1$ + $R_2$ + $R_3$ ne dépasse pas 22 ; tels que la N,N diméthylacrylamide, et la N,N diéthylacrylamide.

**5.** Polymère selon la revendication 4, dans lequel le ou les éventuel(s) monomère(s) hydrophobe(s) (Ib) est(sont) présent(s) en une quantité suffisamment faible pour que le squelette hydrosoluble du polymère soit soluble dans l'eau.

**6.** Polymère selon l'une quelconque des revendications 1 à 5, dans lequel le squelette hydrosoluble est neutralisé totalement ou partiellement par une base minérale ou organique.

**7.** Polymère selon la revendication 6, dans lequel la base est choisie parmi les sels de sodium, ammonium, lithium, calcium, magnésium, ammonium substitué par 1 à 4 groupes alkyle comportant de 1 à 15 atomes de carbone, ou encore parmi la mono, la di- et la tri-éthanolamine, l'aminométhylpropanediol, la N-méthyl-glucamine, les acides aminés basiques, tels que l'arginine et la lysine, et leurs mélanges.

**8.** Polymère selon l'une quelconque des revendications 1 à 7, réticulé totalement ou partiellement.

**9.** Polymère selon la revendication 8, réticulé totalement ou partiellement par un agent de réticulation à polyinsaturation oléfinique utilisé pour la réticulation des polymères obtenus par polymérisation radicalaire, tel que le divinylbenzène, l'éther diallylique, la triallylamine, la tétraallyléthylènediamine, le dipropylèneglycol-diallyléther, les polyglycol-diallyléthers, l'hydroquinone-diallyl-éther, le triméthylolpropane-diallyléther, le tétraallyloxyéthane, les éthers allyliques d'alcools de la série des sucres, les polyallylesters, le tétrallyl-oxéthanoyle ou d'autres allyl- ou vinyléthers alcools polyfonctionnels, le triéthylèneglycol-divinyléther, les esters allyliques de l'acide vinylphosphonique et de l'acide phosphorique, les composés comportant deux ou trois groupes (méth)acrylates ou (méth)acrylamide tels que le diacrylate d'éthylèneglycol, le diacrylate de tétraéthylèneglycol, le diacrylate de butanediol, le méthacrylate d'allyle, le triméthylol propane triacrylate (TMPTA), le méthylène-bis-acrylamide ou leurs mélanges.

**10.** Polymère selon la revendication 8, dans lequel ledit agent de réticulation est le méthacrylate d'allyle, le méthylène-bis-acrylamide, ou le triméthylolpropane triacrylate (TMPTA).

**11.** Procédé selon l'une quelconque des revendications 8 à 10, dans lequel le taux de réticulation est de 0,01 à 10 % en moles, en particulier de 0,2 à 7 % en moles par rapport au polymère.

**12.** Polymère selon la revendication 8, réticulé totalement ou partiellement, en polymérisant, outre, les composants a) et b), un macromonomère à unités à LCST multifonctionnel, par exemple porteur de deux ou trois fonctions insaturées.

**13.** Polymère selon la revendication 12, dans lequel le macromonomère à LCST multifonctionnel est polymérisé dans des proportions permettant d'obtenir un taux de réticulation molaire de 0,01 à 10 %.

**14.** Polymère selon l'une quelconque des revendications 1 à 13, dans lequel le squelette hydrosoluble a une masse molaire de 1 000 g/mole à 50 000 000 g/mole, de préférence de 10 000 g/mole à 10 000 000 g/mole.

**15.** Polymère selon l'une quelconque des revendications 1 à 14, dans lequel le groupe A du ou des macromonomère (s) de formule (II) est choisi parmi les groupes vinyle, allyle, acryle et méthacryle.

**16.** Polymère selon l'une quelconque des revendications 1 à 15, dans lequel les unités à LCST B du macromonomère de formule (II) sont constituées d'un ou plusieurs polymères choisis parmi les polymères suivants :

- les polyéthers tels que le polyoxyde d'éthylène (POE), le polyoxyde de propylène (POP), et les copolymères statistiques d'oxyde d'éthylène (OE) et d'oxyde de propylène (OP) ;
- les polyvinylméthyléthers ;
- les dérivés polymériques et copolymériques N-substitués de l'acrylamide ayant une LCST ; et
- le poly-N-vinylcaprolactame et les copolymères de N-vinyl caprolactame.

**17.** Polymère selon l'une quelconque des revendications 1 à 16, dans lequel les unités à LCST du polymère sont constituées de poly oxyde de propylène $(POP)_n$ où n est un nombre entier de 10 à 50, ou de copolymères statistiques d'oxyde d'éthylène (OE) et d'oxyde de propylène (OP), représentés par la formule $(OE)_m$ $(OP)_n$, dans laquelle m est un nombre entier allant de 1 à 40, de préférence de 2 à 20, de préférence encore de 2 à 10, et n est un nombre entier allant de 10 à 60, de préférence de 20 à 50.

**18.** Polymère selon la revendication 17, dans laquelle la masse molaire des unités à LCST du polymère est de 500 à 5 300 g/mole, de préférence de 1 000 à 4 000 g/mole, et de préférence encore de 1 500 à 3 000 g/mole.

**19.** Polymère selon l'une quelconque des revendications 1 à 16, dans lequel les unités à LCST du polymère sont constituées par un polymère choisi parmi le poly N-isopropylacrylamide, le poly N-éthyl acrylamide et les copolymères de N-isopropylacrylamide ou de N-éthylacrylamide et d'un ou plusieurs monomère(s) (Ia) et (Ib) choisi(s) parmi les monomères, tels que définis dans les revendications 3 et 4.

**20.** Polymère selon la revendication 19, dans laquelle la masse molaire des unités à LCST du polymère est de 1 000 g/mole à 30 000 g/mole.

**21.** Polymère selon l'une quelconque des revendications 1 à 16, dans lequel les unités à LCST du polymère sont constituées par un poly-N-vinylcaprolactame ou un copolymère de N-vinylcaprolactame et d'un ou plusieurs monomère(s) choisi(s) parmi les monomères vinylique(s) (Ia) et (Ib), tels que définis dans les revendications 3 et 4.

**22.** Polymère selon la revendication 21, dans lequel la masse molaire des unités à LCST est de 1 000 à 30 000 g/mole.

**23.** Polymère selon l'une quelconque des revendications 1 à 22, dans lequel la proportion massique des unités à LCST du polymère est de 5 à 70 %, de préférence de 20 à 65 % et mieux encore de 30 à 60 %, par rapport au polymère.

**24.** Procédé de préparation du polymère hydrosoluble selon l'une quelconque des revendications 1 à 23, dans lequel :

- on réalise la copolymérisation radicalaire par précipitation d'un ou plusieurs monomères hydrosolubles (Ia) et, éventuellement, de un ou plusieurs monomères hydrophobes (Ib) en une quantité minoritaire, et d'un ou

plusieurs macromonomères de formule (II) dans un milieu comprenant un alcool et de l'eau ;
- on isole le polymère obtenu.

**25.** Procédé selon la revendication 24, dans lequel ledit alcool est un alcool aliphatique comportant de 1 à 4 atomes de carbone, tel que le méthanol, l'éthanol, le propanol, l'isopropanol et le tertiobutanol.

**26.** Procédé selon la revendication 24, dans lequel le milieu a une teneur en eau inférieure à 10 % en poids, de préférence inférieure à 5 % en poids.

**27.** Procédé selon la revendication 24, dans lequel la copolymérisation radicalaire est réalisée à une température comprise entre -10°C et 100°C, de préférence entre 20°C et 70°C.

**28.** Procédé selon la revendication 24, dans lequel la copolymérisation est réalisée en présence d'un initiateur de copolymérisation générant des radicaux libres.

**29.** Procédé selon la revendication 28, dans lequel ledit initiateur de copolymérisation est choisi parmi l'eau oxygénée ; les composés organiques peroxydés tels que le peroxyde de benzène, l'hydroperoxyde de tertiobutyle, l'hydroperoxyde de méthyléthylcétone ; les composés diazotés tels que l'azobisisobutyronitrile, l'azobisdiméthylvaleronitrile ; les couples oxydant/réducteur tels que peroxodisulfate d'ammonium/métabisulfite de sodium, et peroxodisulfate d'ammonium/N,N,N',N' tétraméthylène diamine.

**30.** Procédé selon la revendication 24, dans lequel la copolymérisation est initiée à l'aide de photoinitateurs.

**31.** Procédé selon la revendication 24, dans lequel la copolymérisation est initiée par une radiation, telle que les rayons UV, les neutrons, ou par plasma.

**32.** Procédé selon la revendication 24, dans lequel la copolymérisation est effectuée en milieu inerte, de préférence sous atmosphère d'azote ou d'argon.

**33.** Composition aqueuse comprenant au moins un polymère tel que défini selon l'une quelconque des revendications 1 à 23, et une phase aqueuse.

**34.** Composition selon la revendication 33, dans laquelle le polymère est présent en une quantité comprise entre 0,01 et 20 % en poids, notamment de 0,05 à 15 % en poids, et en particulier de 0,1 à 10 % en poids.

**35.** Composition selon l'une quelconque des revendications 33 à 34, comprenant par ailleurs un milieu physiologiquement, cosmétiquement ou dermatologiquement acceptable.

**36.** Utilisation cosmétique de la composition selon l'une quelconque des revendications 33 à 35, pour le nettoyage et/ou le maquillage et/ou le soin et/ou la protection solaire des matières kératiniques.

**37.** Procédé de traitement cosmétique pour le soin et/ou le nettoyage et/ou le maquillage et/ou la protection solaire des matières kératiniques, consistant à appliquer sur les matières kératiniques, une composition selon l'une quelconque des revendications 33 à 35.

Office européen des brevets

# RAPPORT DE RECHERCHE EUROPEENNE

Numéro de la demande
EP 02 29 1195

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | US 6 018 033 A (BONGJO SEONG ET AL) 25 janvier 2000 (2000-01-25) * colonne 11, ligne 27-40; revendication 2 * | 1 | C08F290/06 C08F290/04 A61K7/48 A61K7/06 |
| X,D | WO 95 24430 A (ALCON LAB INC ;UNIV WASHINGTON (US)) 14 septembre 1995 (1995-09-14) * exemple 6 * | 1-23 | |
| X,D | EP 1 069 142 A (ICI PLC) 23 mai 1984 (1984-05-23) * revendication 8 * | 1 | |
| X | FR 2 780 422 A (RHONE POULENC CHIMIE) 31 décembre 1999 (1999-12-31) * revendication 1 * | 1 | |
| X | VAMVAKAKI M ET AL: "Synthesis of water-soluble statistical copolymers and terpolymers containing pendent oligo(ethylene glycol derivatives)" POLYMER, ELSEVIER SCIENCE PUBLISHERS B.V, GB, vol. 40, no. 18, août 1999 (1999-08), pages 5161-5171, XP004164984 ISSN: 0032-3861 * le document en entier * | 1 | DOMAINES TECHNIQUES RECHERCHES (Int.Cl.7) C08F A61K |
| A,D | WO 98 48768 A (MENDUM THOMAS H E ;KEARNEY MARIE (US); AHEARN PETER M (US); BROMBE) 5 novembre 1998 (1998-11-05) * revendications * | 1 | |

-/--

Le présent rapport a été établi pour toutes les revendications

| Lieu de la recherche | Date d'achèvement de la recherche | Examinateur |
|---|---|---|
| LA HAYE | 16 septembre 2002 | Meulemans, R |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Office européen des brevets

# RAPPORT DE RECHERCHE EUROPEENNE

Numéro de la demande
EP 02 29 1195

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X,D | HOURDET D ET AL: "Synthesis of thermoassociative copolymers" POLYMER, ELSEVIER SCIENCE PUBLISHERS B.V, GB, vol. 38, no. 10, 1 mai 1997 (1997-05-01), pages 2535-2547, XP004059755 ISSN: 0032-3861 * le document en entier * ----- | 1 | |

| DOMAINES TECHNIQUES RECHERCHES (Int.Cl.7) |
|---|
| |

Le présent rapport a été établi pour toutes les revendications

| Lieu de la recherche | Date d'achèvement de la recherche | Examinateur |
|---|---|---|
| LA HAYE | 16 septembre 2002 | Meulemans, R |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 02 29 1195

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

16-09-2002

| Document brevet cité au rapport de recherche | | Date de publication | Membre(s) de la famille de brevet(s) | | | Date de publication |
|---|---|---|---|---|---|---|
| US 6018033 | A | 25-01-2000 | AU | 7478498 | A | 08-12-1998 |
| | | | WO | 9851694 | A2 | 19-11-1998 |
| WO 9524430 | A | 14-09-1995 | AT | 206441 | T | 15-10-2001 |
| | | | AU | 692852 | B2 | 18-06-1998 |
| | | | AU | 2093295 | A | 25-09-1995 |
| | | | CA | 2184814 | A1 | 14-09-1995 |
| | | | CN | 1145080 | A | 12-03-1997 |
| | | | DE | 69523044 | D1 | 08-11-2001 |
| | | | DE | 69523044 | T2 | 20-06-2002 |
| | | | EP | 0748342 | A1 | 18-12-1996 |
| | | | JP | 10500148 | T | 06-01-1998 |
| | | | WO | 9524430 | A2 | 14-09-1995 |
| EP 1069142 | A | 17-01-2001 | DE | 10029462 | A1 | 03-01-2002 |
| | | | BR | 0002793 | A | 13-03-2001 |
| | | | EP | 1069142 | A1 | 17-01-2001 |
| | | | JP | 2001081148 | A | 27-03-2001 |
| FR 2780422 | A | 31-12-1999 | FR | 2780422 | A1 | 31-12-1999 |
| | | | AU | 4271899 | A | 17-01-2000 |
| | | | BR | 9911567 | A | 20-03-2001 |
| | | | CN | 1312830 | T | 12-09-2001 |
| | | | EP | 1095079 | A1 | 02-05-2001 |
| | | | WO | 0000528 | A1 | 06-01-2000 |
| WO 9848768 | A | 05-11-1998 | AU | 7174998 | A | 24-11-1998 |
| | | | CA | 2259464 | A1 | 05-11-1998 |
| | | | EP | 0927019 | A1 | 07-07-1999 |
| | | | WO | 9848768 | A1 | 05-11-1998 |

EPO FORM P0460

Pour tout renseignement concernant cette annexe : voir Journal Officiel de l'Office européen des brevets, No.12/82